# EUROPEAN PATENT APPLICATION

(11) **EP 4 407 297 A1**
(43) Date of publication of application: **31.07.2024**
(21) Application number: 24153572.3
(22) Date of filing: 24.01.2024
(51) Int. Cl.: G01N 21/27, G01N 21/31, G01N 21/41, G01N 21/53, G01N 21/552, G01N 21/85, G01F 1/7086, G01F 1/74, G01N 33/543, G01F 1/704, G01F 23/292, G01N 15/00, G01N 35/10, G01N 21/84

(54) **FLUID ANALYSIS USING OPTICAL SYSTEMS**

(30) Priority: 24.01.2023 LU 103065
(71) Applicant: Stratec SE, 75217 Birkenfeld (DE)
(72) Inventor: Rech, Thomas, 75217 Birkenfeld (DE); Schmalz, Tobias, 75217 Birkenfeld (DE)
(74) Representative: Tegethoff, Sebastian

(57) **Abstract**

The disclosure relates to a method for analysing parameters of a fluid in a hose (5), comprising the steps of calibrating a current of a light source (20) for emitting light (10) so that the signals resulting from light received by a receiver (30) arranged behind an air filled hose (5) and a fluid filled hose (5) allow a differentiation between the signal for an air filled hose (5) and a fluid filled hose (5); emitting light (10) by the light source (20) at a defined wavelength through the hose (5) wherein the receiver (30) is arranged behind the hose (5) with an angle with respect to the axis of a light beam (10); detecting a phase boundary between air and fluid during aspiration of the fluid into the hose (5) by measuring signals with the receiver (30); determining when the hose (5) is filled with fluid; increasing the current of the light source (20) when the hose (5) is filled with fluid; measuring absorption of the light (10) and/or scattered light (10) with the receiver (30); and evaluating the amount and intensity of the measured absorbed and/or scattered light (10).

## Description

### Field of the Disclosure

The disclosure relates to a method for analysing parameters of a fluid in a hose.

### Brief description of the related art

Devices for use in clinical diagnostics and life sciences are produced by a number of companies. For example, STRATEC^{®} SE, Germany, manufactures numerous devices for diagnostic specimen handling and detection for use in automated analyser systems and other laboratory instrumentation.

So called samples will be provided to said systems for their analysis. The analysis requires the processing of the samples by adding or removing fluids which may comprise solids like magnetic beads or other essential specific substances. Whatever is required for a successful analysis of a sample will have to be present in a pre-defined concentration. In other words, the analysis will only provide correct results if the required components were present in the respective step in a correct concentration.

Measuring and determining the concentration of a respective compound in a running analysis will not only lead to correct results, but it will also reduce the time for the analysis because there are no additional steps required for determining the presence or concentration of a component.

The published U.S. patent application US 2008/0192255 A1 discloses a device and a method which are used for an optical detection of at least one phase transition between at least two media, which are taken into a line and/or dispensed from the line by an intake and/or dispensing device. A light-emitting transmitter emits light across and onto the line at a measurement point provided for this purpose. A receiver receives the emitted light, which is influenced by media in the line, to form reception signals. At least one waveguide, which is arranged up to the measurement point on the probe, is provided between the transmitter and/or the receiver and the measurement point (M). Due to the fact that the waveguide is arranged in parallel to the line at least in the area near the probe and at least one deflection lens is provided in the area of the measurement point to deflect the light emitted and/or the light to be detected, a phase boundary or state can be detected, and the dead volume is reduced. Device and method of US 2008/0192255 A1 do only relate to the detection of at least one phase transition. The analysis of further properties of a medium would require additional components and/or further steps for the analysis. A disadvantage of the disclosed device and method relates to the requirement of a defined measuring point and that only phase transitions can be detected by the device.

Published International application WO 2018/005213 A1 discloses apparatus includes a pipe through which a multiphase fluid flows, with a transparent window structure formed in the pipe. A collimated light source emits light through the transparent window structure into the pipe having a wavelength at which a component of a desired phase of the multiphase fluid is absorptive. A photodetector is positioned such that the emitted light passes through the multiphase fluid in the pipe to impinge upon the photodetector. The photodetector has an actual dynamic range for collimated light detection. Processing circuitry is configured to adjust a power of the collimated light source dependent upon an output level of the photodetector so as to cause measurement of the emitted light over an effective dynamic range greater than the actual dynamic range. Properties of the multiphase fluid are determined as a function of the measured emitted light.

Published Japanese patent application JP 2013 113652 A relates to an air bubble detector which detects air bubbles in liquid stored in a nozzle chip consisting of a translucent material includes: a light emission part which irradiates light with the same wavelength as that of absorption peak wavelength of water toward the nozzle chip; a light receiving part which is installed on the opposite side of the light emission part on both sides of the nozzle chip, and detects light volume of the light passed through the nozzle chip; a Z driving part which relatively moves the nozzle chip in the axis direction; and a control part which controls drive of the Z driving part, and determines presence/absence of the air bubbles on the basis of variation of transmitted light volume in accordance with relative movement of the nozzle chip in the axis direction. It appears disadvantageous that the nozzle chip requires components for its movement which makes the detector structurally complex.

Published Canadian patent application CA 2 254 690 A1 discloses a bubble detector/direction sensor for detecting the presence of liquid and gas in a stream of liquid and gas segments flowing through an elongated transparent tube and for sensing the direction of flow of the stream. The sensor has first and se cond light sources, first and second input fiber optic bundles, and first and second collection fiber optic bundles. The first and second input fiber optic bundles c ouple the first and second light sources to first and second sides of the tube, respec tively, so that light passes through the tube. When the tube is filled with liquid, the light passing therethrough from each light source passes through first and second predetermined regions. When the tube is filled with gas, the light passing therethrough passes outside of the predetermined regions. The first and second collection fiber optic bundles are coupled to the second and first sides of the tube, respectively, outside of the predetermined regions. A circuit is provided for producing signals indicating the presence of liquid in the tube, the presence of gas in the tube, and the direction of flow of liquid and gas through the tube based upo n the light received by the first and second collection fiber optic bundles. In altern ative embodiments, the sensor includes an additional collection fiber optic bundle cou pled to the second side of the tube that is positioned within the first predetermined region. In these embodiments, the circuit produces a signal indicating the presence of a liquid in the tube containing a marker dye having known absorbance characteristi cs based upon the light received by each of the collection fiber optic bundles and the known absorbance characteristics. The use of two light sources and two detectors makes the sensor according to this document structurally complex.

Published U.S. patent application US 5,447,692 discloses a single detector to monitor several process functions, e.g. reaction efficiency, reagent flow rates, the presence of empty reagent reservoirs, the absence of a chemical reactor column in the system, blockage of flow system, etc., at a single location in the flow system. In accordance with the detection scheme of the disclosure, only one detector is required to accomplish the same, if not more, functions as many detectors in the prior art instruments. In the described embodiment of this document, an optical detector is positioned downstream of a chemical reaction chamber. This detector monitors the effluent from the reaction chamber to monitor the reaction efficiency. It also monitors the system functions upstream of the reaction chamber. System flow rate is monitored by detecting the presence of a gas bubble which has been introduced into the system at a known instance. Depletion of reagents in the reservoirs can be detected by monitoring the absence of the reagents at the detector at times when the reagents are expected. The flow system may be periodically diagnosed to check for flow blockage or missing flow component, by monitoring the flow past the detector which corresponds to a predetermined succession of reagents introduced into the system. Any deviation from a predictable succession of changes in the flow monitored by the detector indicates possible blockage of the flow delivery system or missing flow components.

Published European patent application EP 1 775 574 A1 discloses a method of determining a kind of a sample, in a method for analysing a substance by the steps of supplying the sample to be analysed to a reaction system by a supplying means comprising a transparent region composed of a transparent material, reacting a reagent or detecting the substance with the sample in the reaction system, and analysing a signal derived from a product obtained by the reaction, characterized by irradiating the transparent region with light in the supplying step, and analysing an optical intensity of the light.

There is a need for a system and method allowing the analysis of different properties of a fluidic sample.

### Object of the Disclosure

It is therefore the object of this disclosure to provide a system and a method for determining different properties of a fluidic sample.

### Summary of the Disclosure

The present disclosure provides a method for analysing parameters of a fluid in a hose, comprising the steps of:
- Calibrating a current of a light source for emitting a light beam so that the signals resulting from the light beam received by a receiver arranged behind a hose allow a differentiation between the signals received from an air-filled hose and a fluid-filled hose;
- Emitting the light beam by the light source at a defined wavelength through the hose wherein the receiver is arranged behind the hose with an angle with respect to the axis of the light beam;
- Detecting a phase boundary between air and fluid during aspiration of the fluid into the hose by measuring signals with the receiver;
- Determining when the hose is filled with fluid;
- Increasing the current of the light source when the hose is filled with fluid;
- Measuring absorption of the light and/or scattered light with the receiver; and
- Evaluating the amount and intensity of the measured absorbed and/or scattered light.

In an embodiment of the method, the light source may comprise at least one LED.

Another embodiment of the method according to the present disclosure relates to the use of an additional sensor for detecting a transmitted light beam and its wavelength during measuring absorption.

The method of the present disclosure may further comprise the step of evaluating the amount of a scattered light beam and comparing the determined amount of the scattered light beam with an amount of the scattered light beam relating to a known concentration of a first compound in the fluid.

The first compound can be selected from the group comprising magnetic beads, antibodies, proteins, peptides, nucleic acids and synthetic or biological substances.

It is further envisaged that evaluating the amount of an absorbed light beam comprises comparing the measured absorption with a known absorption relating to a known concentration of a second compound, wherein the second compound can be a dye.

The method of the present disclosure may also comprise measuring the absorbed light beam and/or the scattered light beam with the light beam at different wavelengths.

Another aspect of a method according to the present disclosure relates to the measurements with the light beam at a wavelength having 660 nm and 500 nm.

Still other aspects, features, and advantages of the present disclosure are readily apparent from the following detailed description, simply by illustrating preferable embodiments and implementations. Accordingly, the drawings and descriptions are to be regarded as illustrative in nature, and not as restrictive. Additional objects and advantages of the disclosure will be set forth in part in the description which follows and in part will be obvious from the description, or may be learned by practice of the disclosure.

### Summary of the Figures

The disclosure will be described based on figures. It will be understood that the embodiments and aspects of the disclosure described in the figures are only examples and do not limit the protective scope of the claims in any way. The disclosure is defined by the claims and their equivalents. It will be understood that features of one aspect or embodiment of the disclosure can be combined with a feature of a different aspect or aspects of other embodiments of the disclosure, in which:
FIG. 1 shows an air-filled hose where almost 100% of the emitted light from a light source is reflected at the inner surface of the hose and hits the receiver.
FIG. 2 shows a fluid filled hose where almost no reflection of the light occurs.
FIG. 3 shows the dependency of the optical light value output from the applied LED current.
FIG. 4 shows absorption measurements at 660nm and 500nm for determining the concentration of red blood cells in a fluid.

### Detailed Description of the Disclosure

The technical problem is solved by the independent claims. The dependent claims cover further specific embodiments of the disclosure.

The present disclosure relates to performing various analyses directly in a pipettor while using the existing optical sensor technology for the following tasks:
1. Checking the concentration of magnetic particles in a suspension: If the concentration of the magnetic particles is below certain values, this may strongly influence the significance of subsequent analyses. Determining the bead concentration is therefore used as a kind of process control and allows the process to be stopped before the actual analysis, or at least the test concerned to be flagged as potentially faulty.
2. Checking the concentration of a specific test reagent in an expected concentration range: It will be possible to determine if a reagent has been significantly concentrated by evaporation or diluted by condensed water or system fluid from the pipettor. The determination of the reagent concentration thus serves also as a kind of process control and enables the process to be stopped before the actual analysis, or at least to mark the test concerned as potentially faulty.
3. Detecting whether blood plasma samples are haemolytic, icteric, or lipemic: HIL samples strongly influence the analysis of a sample, therefore the affected samples can be discarded or marked after HIL determination.
4. Checking the concentration of red blood cells in a specific concentration range: It will be possible to determine the amount of the required reagents for each sample. Since the concentration of red blood cells varies greatly between samples, this test allows to estimate the necessary dilution for each sample beforehand. In this case, it is more than just a process control. The aim here is not to stop the process or to mark the samples, but to intervene in the process and to adjust the subsequent dilution accordingly.

A fluid within the meaning of the present disclosure shall designate a liquida solid, or mixtures thereof.

The disclosure relates to the use of optical sensor technology to quantify various parameters of the fluid by means of scattered light and absorption measurement. Individual measurements can be performed with the existing hardware. A hardware change may be necessary if the colour of the sample is to be determined.

The present disclosure will now be explained in greater detail by way of example, with reference to the accompanying drawings. However, the exemplary embodiments pertain only to examples. The following drawings show the operating principle of the integrated optical sensor system. This sensor system works on the principle of total reflection to distinguish a fluid inside the hose from air.

In the case of an air-filled hose, the light emitted by the transmitter is reflected at the inner surface of the hose which corresponds to a total reflection according to the refractive index of the hose. The light thus hits the receiver, which is arranged at a suitable angle.

If a fluid is present in the tube, however, the light from the transmitter passes undisturbed straight through the tube and therefore does not hit the receiver. In this case, the refractive index of the hose and the fluid is approximately identical.

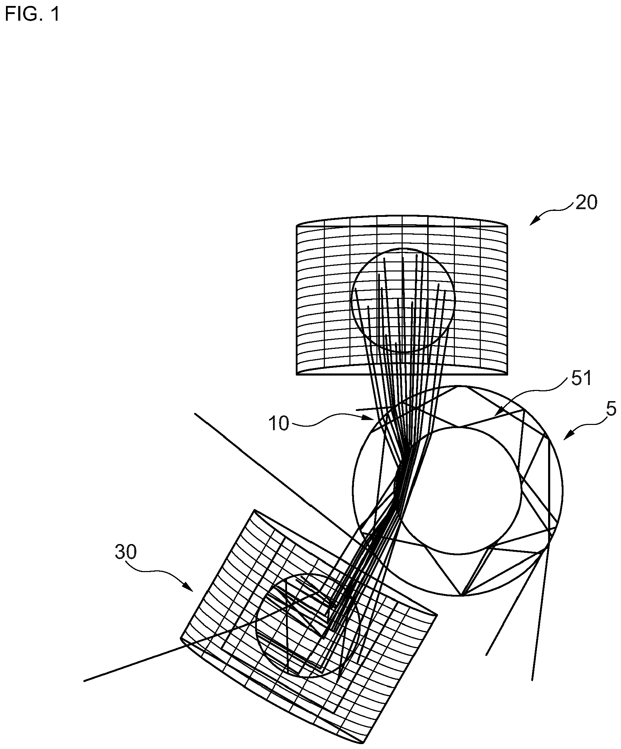

FIG. 1 shows an air-filled hose 5 where almost 100% of the emitted light 10 from a light source 20 is reflected at the inner surface 51 of the hose 5 and hits the receiver 30. A high signal level is detected indicating that the hose is filled with air.

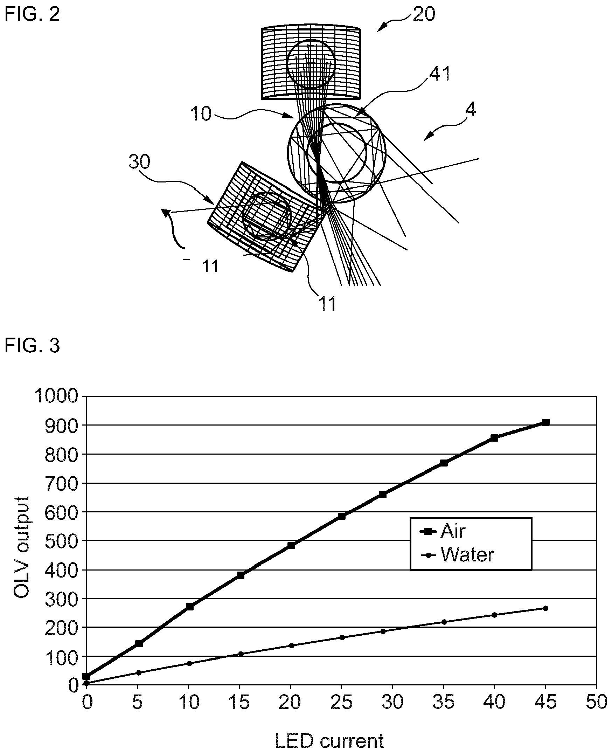

FIG. 2 shows a fluid filled hose 4. There is almost no reflection of the emitted light 10 from a light source 20 on the inner surface 41 of the hose 4 when the hose is filled with fluid so that most of the light does not reach the receiver 30. A low signal level is detected indicating that hose is filled with liquid. This method works independently from the colour of the fluid.

Even in a fluid filled hose a small part 11 of the light 10 also reaches the receiver 30 due to tolerances, although the light has not been reflected at the inner surface of the hose but has previously penetrated the fluid. This part of light reaching the receiver is unintended, but it has been discovered that it may serve as a basis for the absorption measurements according to the present disclosure. In addition, the light is also partly scattered by particles in the fluid and thus also reaches the receiver.

During normal operation, i.e., during detecting air or fluid in the hose, both effects are quite small and are practically lost in the background noise. However, both effects are always present, and depending on the fluid, sometimes one predominates, sometimes the other.

It has been discovered that for using the described effect of unintended reflection for the analysis of the liquid, the following steps must be performed:
- Calibration of the sensor system for standard air or fluid detection in such a way that the LED current, and thus the LED brightness of the light source, is configured in a manner that the signal distance between the air and the fluid values has a difference allowing a clear differentiation between the values. A low LED current will help to protect the LEDs, but the current has to be high enough to allow a clear differentiation between air and fluid in the hose FIG. 3 illustrates a measurement of an optical light value output depending on the applied current to the LED. In the example of FIG. 3 a current of 25 appears to be sufficient for a reliable differentiation between air and fluid.
- Calibration of the sensor system for the extended analysis: The LED current is adjusted in such a way that the receiver is not overdriven at the maximum expected scattered light or at the maximum expected light of an absorption measurement. A suitable fluid/solution is required for the calibration, which means that the fluid used for calibration has comparable properties than the fluid which is used in the analysis.
- The LED current for a phase boundary detection is usually much lower than for fluid analyses. Usually, the necessary LED current for the concentration measurement is more than 10 times higher.
- If different analyses (e.g. magnetic particle concentration and blood concentration) are to be performed with the same system, the calibration step must be performed separately for each kind of analysis. The calibration solution must also be selected in a manner that it has comparable properties to fluids which are used in the respective analysis.
- Detection of the phase boundary during aspiration of the fluid. The LED current is thereby set to the previously determined (low) value.
- Subsequently, when it is known on the basis of the previous measurement that there is fluid in the pipettor, the LED current for analyzing the fluid is increased to the value determined for the analysis during calibration.
- The process is practically not delayed by this, as measurements are only taken for a few milliseconds.
- Analysis of the fluid:
   - A combination of scattered light and absorption is always measured, or it is not possible to distinguish where the light comes from.
   - To evaluate the analysis result correctly, i.e. whether a colour of the fluid or its particle concentration is measured, prior knowledge about the liquid must be available. The system must know which fluid it expects.

The state-of-the-art system uses a broadband receiver for detecting a range between 380nm to 1100nm and a green LED (523nm). For red coloured fluids, like blood, the system works very well, because the green light is absorbed very well by blood and other red coloured fluids.

If other colours are to be detected or measured, it may be necessary to adjust the colour which the LED emits. In addition, the simple broadband receiver could be replaced by a colour resolving sensor or spectrometer, then possibly in combination with a white LED.

A further improvement, especially regarding the absorption measurement, can be achieved by adding another sensor in the transmitted light range (the range in which the majority of the light radiates in the case of a liquid-filled tube), thereby improving the accuracy of the absorption measurements.

In principle, the original function for the detection of air or fluid remains the same for all variants, despite possible required hardware changes.

Checking the magnetic particle concentration is related to the physical effect of scattered light. The aim to be achieved is to determine whether the concentration of the magnetic particles is within a range suitable for the subsequent sample analysis.

A faulty magnetic particle concentration can be caused, for example, by a faulty batch of magnetic particle reagent or by settling of the magnetic particles in the container. Therefore, magnetic particle reagent containers are usually moved in the instrument, e.g. by rotation, for preventing the settling of the magnetic particles. Magnetic particles that have accumulated or settled on the bottom of the container are no longer available for the pipettor access, so that the concentration in the solution decreases.

It is therefore possible to detect faulty magnetic particle solutions and/or faulty "stirring" of the magnetic particle solution. No hardware changes are necessary.

Determining a reagent concentration is related to a colour measurement or the measurement of absorption. It is checked whether the concentration of a specific test reagent is within a certain range. A decrease in concentration indicates dilution, e.g. by condensation or system fluid. An increase in concentration may be caused by evaporation.

If the reagent concentration deviates from a required concentration or range of a concentration, either a warning can be issued to control the system, or the amount of reagent used for sample analysis can be adjusted according to the required concentration. A suitable dye with a known concentration must be used that does not interfere with the actual reaction or sample analysis. In the current system, for instance a LED light source with a peak wavelength of 523 nm can be installed in combination with the use of red dyes related to a high absorption in green light.

Transmitting LED and dye must match each other within the meaning of a high absorption of the LED wavelength in the dye. Either the adaptation of the dye to the existing hardware, or the adaptation of the hardware to the dye or its absorption is necessary, respectively.

Determining the concentration test of red blood cells is based on the mixture of scattering and absorption effects. Due to the interaction of absorption and scattering effects on the red blood cells, the measurement in only one colour channel is not unambiguous over the complete concentration range from 0% to 100%. Therefore, the determination of the concentration of red blood cells for the complete concentration range is only possible using different wavelengths or colours by either using at least two differently coloured LEDs as light sources, e.g. green and red, or by using a white LED and a spectrally resolving sensor.

If only one light colour is used, only a limited measuring range is available. By comparing the colour values from the red channel at approximately 660nm and the green channel at approximately 500nm, it is possible to determine the concentration of red blood cells from 100% to approximately less than 1%.

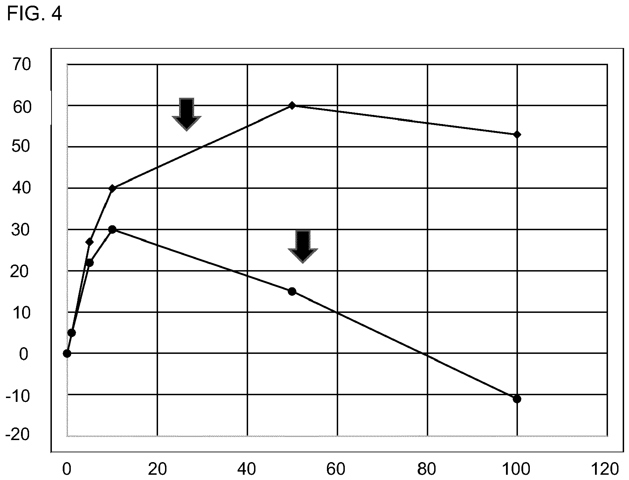

FIG. 4 shows the results from such measurements at 660nm (upper line with squares) and 500nm (lower line with circles). A clearly linear increasing absorption depending on the concentration of red blood cells can be observed at 660nm for a concentration between 0 and 50%. Which is indicated with the arrow. At 500nm a clearly linear decreasing absorption can be observed for a concentration between 10 and 100% red blood cells, which is also indicated with an arrow.

Basically, it is possible to determine the concentration of red blood cells with limited measuring range of concentration with the existing hardware. An improvement of the measuring accuracy or an extension of the measuring range requires hardware changes related to the LED wavelength of the light source and possibly of the colour sensor or spectrometer.

In the case of haemolytic, lipemic or icteric blood samples (HLL samples), the appearance and thus the optical behaviour of the blood plasma - which is normally yellowish-clear and transparent - changes, so that an optical analysis of the sample is impaired. The reasons vary and may be partly caused by improper handling or blood sampling, but may also indicate pathological changes in the blood. Haemolytic samples can be caused by malaria, for example.

Normally, such faulty samples must be sorted out manually by the user or laboratory personnel before loading them to the analysis system. However, since this is not always done, it is helpful if the instrument can detect and sort out such samples itself.

Haemolytic samples have a reddish clear staining of the plasma so that a detection via a colour and absorbance measurement is possible.

Icteric samples have a dark yellow, brown or greenish coloration of the plasma, so that a detection via colour and absorption measurement is possible.

Lipemic samples are milky turbid so that a detection by scattered light measurement is possible.

The marking of plasma samples is a combination of scattered light and absorbance measurement. A calibration can be performed by comparing different turbidity and absorbance values to normal samples. An algorithm detects whether the sample is lipemic, icteric, or haemolytic by comparing normal values to the measured values. If a specific pattern is detected, a warning is issued to the user and the process is adapted to the new sample type.

If the LED wavelength is selected appropriately, it is possible to work with one LED colour and a simple broadband receiver. The LED wavelength must be well absorbed by both haemolytic and icteric samples. In this case the measured value would be in the middle range for a normal or good samples. Haemolytic and icteric samples would decrease the measured value due to higher absorption values while lipemic samples would increase the measured value due to existing scattered light caused by turbidity. It is not possible to distinguish haemolytic and icteric samples from each other - which is usually not necessary - but it is possible to distinguish HIL samples from normal samples.

If it is also required to distinguish haemolytic from icteric samples and possibly also determine the degree of change, multi-channel measurements using several colour channels will be necessary. Either via white LED and a spectral sensor or a plurality of LED colours and a broadband receiver may be used which means that a hardware modification is necessary.

The advantages of the disclosure can be summarized as follows:
- Checking the concentration of magnetic particles in suspension:
   - Process control whether absolutely sufficient magnetic particles are present in the solution or whether sufficient magnetic particles are freely present in the solution (i.e. they have not settled).
- Checking the concentration of a specific test reagent in an expected concentration range.
   - Process control allowing an error message to control the system.
   - Adjusting the amount of reagent for sample analysis. This makes it possible to dose expensive reagents more accurately and thus save costs.
- Checking the concentration of red blood cells and either marking the sample or adjusting the subsequent process/dilution.
   - Both process control and process control (adjustment of dilution) possible.
- Detecting whether blood plasma samples are hemolytic, lipemic, or icteric.
   - Marking/sorting out of these samples.

The foregoing description of the preferred embodiment of the disclosure has been presented for purposes of illustration and description. The embodiment was chosen and described in order to explain the principles of the disclosure and its practical application to enable one skilled in the art to utilize the disclosure in various embodiments as are suited to the particular use contemplated. It is intended that the scope of the disclosure be defined by the claims appended hereto. The entirety of each of the aforementioned documents is incorporated by reference herein.

### Reference Numerals

- 4: fluid filled hose
- 5: air filled hose
- 10: light
- 20: source
- 30: receiver
- 51: inner surface

## Claims

1. A method for analysing parameters of a fluid in a hose, comprising the steps of:
- Calibrating a current of a light source for emitting a light beam so that the signals resulting from the light beam received by a receiver arranged behind a hose allow a differentiation between signals received from an air-filled hose and a fluid-filled hose;
- Emitting the light beam by the light source at a defined wavelength through the hose wherein the receiver is arranged behind the hose with an angle with respect to the axis of the light beam;
- Detecting a phase boundary between air and fluid during aspiration of the fluid into the hose by measuring signals with the receiver;
- Determining when the hose is filled with fluid;
- Increasing the current of the light source when the hose is filled with fluid;
- Measuring absorption of the light and/or scattered light with the receiver; and
- Evaluating the amount and intensity of the measured absorbed and/or scattered light.

2. The method of claim 1, wherein the light source comprises at least one LED.

3. The method of claim 1 or 2, wherein an additional sensor for detecting a transmitted light beam and its wavelength is used during measuring absorption.

4. The method of any one of claims 1 to 3, comprising the step of evaluating the amount of a scattered light beam and comparing the determined amount of the scattered light beam with an amount of the scattered light beam relating to a known concentration of a first compound in the fluid.

5. The method of claim 4, wherein the first compound is selected from the group comprising magnetic beads, antibodies, proteins, peptides, nucleic acids and synthetic or biological substances.

6. The method of any one of claim 1 or 2, wherein evaluating the amount of an absorbed light beam comprises comparing the measured absorption with a known absorption relating to a known concentration of a second compound.

7. The method of claim 6, wherein the second compound is a dye.

8. The method of any one of claims 1 to 7, wherein measuring the absorbed and/or the scattered light beam is performed with the emitted light beam at different wavelengths.

9. The method of claim 8, wherein the measurements are performed with the emitted light beam at a wavelength having 660 nm and 500 nm.
